# EUROPEAN PATENT SPECIFICATION

(11) **EP 2 603 206 B1**
(45) Date of publication and mention of the grant of the patent: **02.11.2016**
(21) Application number: 11746215.0
(22) Date of filing: 08.08.2011
(51) Int. Cl.: A61K 31/4439, A61K 9/16, A61K 9/20, A61K 9/50

(54) **PHARMACEUTICAL COMPOSITIONS OF METABOTROPIC GLUTAMATE 5 RECEPTOR (MGLU5) ANTAGONISTS**
PHARMAZEUTISCHE ZUSAMMENSETZUNGEN ENTHALTEND ANTAGONISTEN DES METABOTROPISCHEN GLUTAMAT 5 REZEPTORS (MGLU5)
COMPOSITIONS PHARMACEUTIQUES CONTENANT DES ANTAGONISTES DES RÉCEPTEURS MÉTABOTROPIQUES DU GLUTAMATE 5 (MGLU5)

(30) Priority: 11.08.2010 US 372693 P
(43) Date of publication of application: 19.06.2013
(60) Divisional application: 14174022.5
(73) Proprietor: F.Hoffmann-La Roche AG, 4070 Basel (CH)
(72) Inventor: CHATTERJI, Ashish, East Brunswick New Jersey 08816 (US); HUANG, Jingjun, Monmouth Junction New Jersey 08852 (US); KOENNINGS, Stephanie, CH-4103 Bottmingen (CH); LINDENSTRUTH, Kai, 79539 Loerrach (DE); SANDHU, Harpreet K., West Orange New Jersey 07052 (US); SHAH, Navnit Hargovindas, Clifton New Jersey 07012 (US)
(74) Representative: Poppe, Regina
(86) International application number: PCT/EP2011/063602
(87) International publication number: WO 2012/019989

(56) References cited:
- WO-A1-2005/118568
- WO-A1-2008/074697
- US-A- 4 968 508
- TATAVARTI ADITYA S ET AL: "Influence of methacrylic and acrylic acid polymers on the release performance of weakly basic drugs from sustained release hydrophilic matrices", JOURNAL OF PHARMACEUTICAL SCIENCES, vol. 93, no. 9, September 2004 (2004-09), pages 2319-2331, XP002676237, ISSN: 0022-3549
- CHARLES VESEY: 'SURELEASE Application Data Stability of a Sparingly Soluble BCS Class I API Ethylcellulose Coated Multiparticulate' CONTROLLED RELEASE SOCIETY MEETING 2010 OREGON 14 July 2010, pages 1 - 5, XP055148759

## Description

The present invention provides a multi-particulate modified release composition for a poorly water soluble drug which comprises the compound 2-chloro-4-[1-(4-fluoro-phenyl)-2,5-dimethyl-1H-imidazol-4-ylethynyl]-pyridine and pharmaceutically acceptable salts thereof, a rate-controlling polymer, and a pH responding polymer.

The composition comprises the form of a matrix pellet.

The mGlu5 antagonists can exist in an amorphous form, a solvate or form a solid dispersion, co-crystal, or complex with other ingredients.

Many chemical entities are poorly water soluble and possess a pH-dependent solubility. This poor solubility raises significant hurdles in developing a reproducible drug PK profile, which in turn affects the *in vivo* efficacy and safety of the drug.

There are several technical difficulties in the development of poorly soluble, weakly basic compounds. These difficulties include dose dumping due to high solubility of the compound in gastric fluid. Poor solubility and inadequate dissolution rate in the intestine result in lower absorption and bioavailability. Poor solubility also results in high inter and intra subject variability in the pharmacokinetics requiring a wider safety margin. Further, the effect on bioavailability and PK profiles complicates the dosing regimen.

Several modified release technologies are known, such as matrix tablet, pellet, osmotic pump, etc. These technologies were developed mainly for controlled delivery of water-soluble compounds. However, they often prove inadequate for poorly soluble or practically insoluble drugs because of their low solubility and variability in their release in the GI tract.

The emergence of newer therapeutic agents and a growing understanding of both pharmacokinetics and the physiological needs of patients make the task of controlled drug delivery more complex. For example, for poorly water soluble, weakly basic compounds with highly pH dependent solubility, there has been very limited success in providing adequate enhancement of a reproducible drug plasma profile within the therapeutic window. The limited success observed with these approaches was mainly related to a highly pH dependent solubility profile and an extremely low solubility in physiological intestinal fluids. The success of controlled delivery of this type of compound depends on improvement of drug release rate in intestine fluid, a pH-independent release profile in both gastric and intestinal fluid, and minimum inter and intra variation in drug release/absorption among subjects.

Several drug delivery technologies have been developed to address these concerns. Each of these technologies has certain detriments to the development of a drug composition having a pH independent dissolution.

One such method applies a delayed release enteric polymer coating to reduce dose dumping. Generally, this approach applies a thick layer of enteric polymer to delay the release of drug until reaching the intestinal tract. The high solubility of the drug in the low pH gastric fluid provides the strong driving force for drug dissolution and diffusion. However, this approach results in local initiation, fast absorption, high Cmax, and CNS side effects. The problem associated with this technology is an unpredictable PK profile due to inter and intra variation in gastric transit time and food effect.

Another composition strategy to provide pH independent drug release of a weakly basic drug in the GI tract is to incorporate organic acids as microenviromental pH modifiers. For example, the pH-independent release of fenoldopam from pellets with insoluble film coats has been shown. However, these compositions present several issues, such as salt conversion, control of diffusion of small molecular weight acidic pH modifiers, and potential interaction of organic acids with membranes that result in sigmoidal release profiles.

Due to their poor solubility in intestinal fluid, the absorptionlbioavailability of some compounds is dissolution rate limited. Reduction in particle size can improve the dissolution rate, which can provide better absorption potential and likely improved therapeutics. Wet milling and nano-technology are two techniques that can be applied to poorly water-soluble drugs. Formation of a salt, co-crystal, solid dispersion, solvate, or amorphous form increases kinetic solubility of the compound, which provides a higher concentration gradient for drug release. Size reduction and drug form modification are technologies that only reduce inter and intra variation and food effect to a limited extent. pH will still have a big impact on the solubility and dissolution rate of the compound, especially for poorly water soluble, basic compounds.

Controlling drug release by combining polymers has been demonstrated in the literature; however, these systems are designed to provide a zero order release profile. Furthermore, release rate is sensitive to the pH-dependency of the drug's solubility. Such systems do not have a means to increase the dissolution rate at higher pH.

The present compositions are useful for the treatment of CNS related disorders, including treatment resistant depression (TRD) and Fragile-X syndrome.

### Brief Description of the Drawings

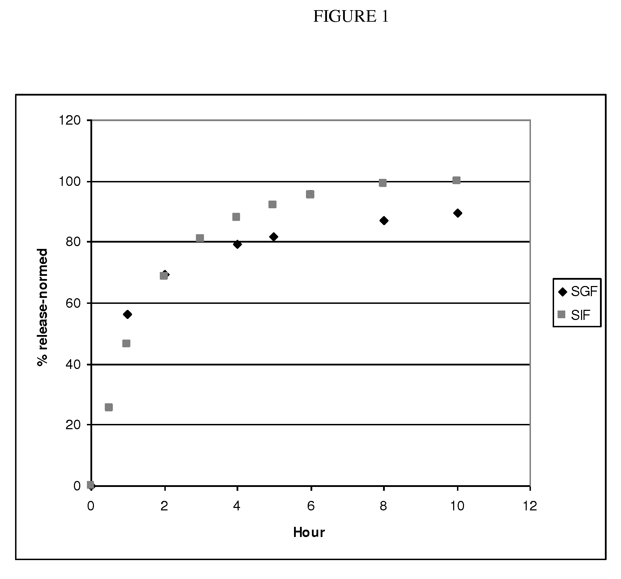

Figure 1 is the *in vitro* dissolution profile of the matrix pellet composition of Example 1 in simulated gastric fluid (SGF) and simulated intestinal fluid (SIF).

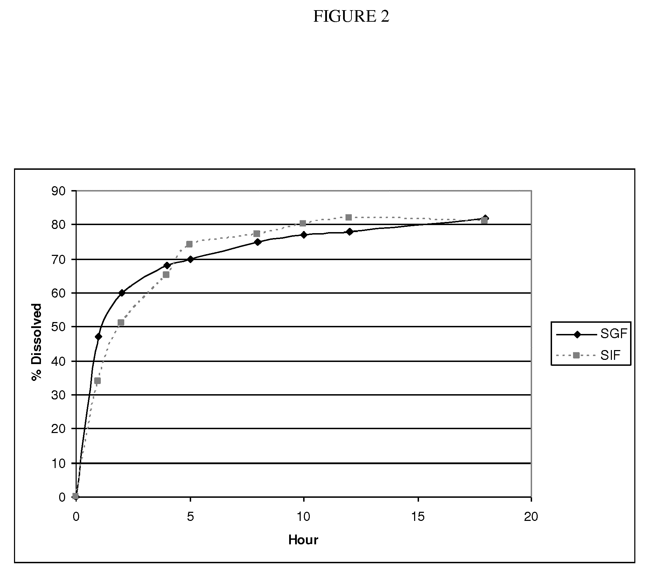

Figure 2 is the *in vitro* dissolution profile of the matrix pellet composition of Example 2 in simulated gastric fluid (SGF) and simulated intestinal fluid (SIF).

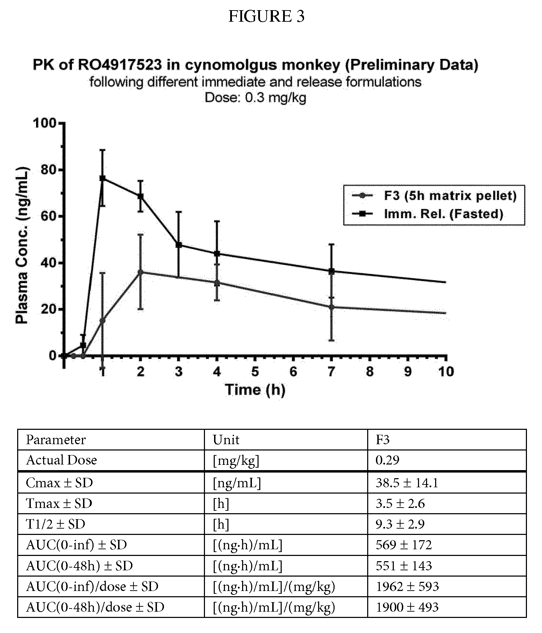

Figure 3 is the *in vivo* intrinsic dissolution PK profile of the composition in Example 3 (F3).

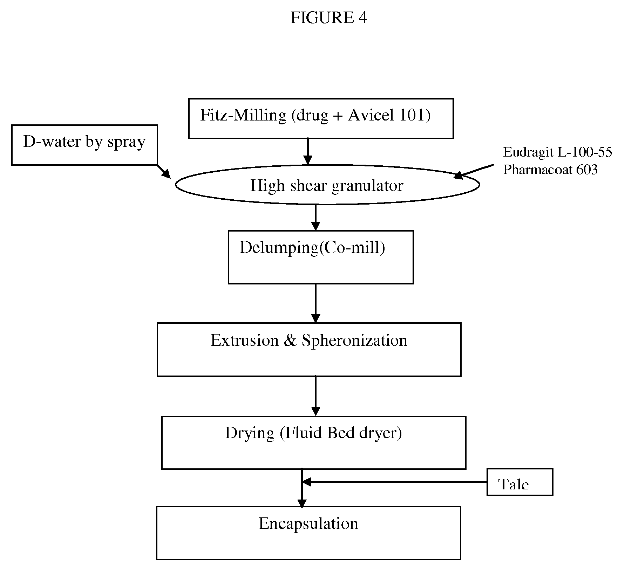

Figure 4 is a flow diagram depicting the process for preparing matrix pellet compositions disclosed herein.

The composition described herein is a modified release technology that provides pH independent delivery of poorly water soluble drugs, particularly the metabotropic glutatmate 5 receptor (mGlu55) antagonist 2-chloro-4-[1-(4-fluoro-phenyl)-2,5-dimethyl-1H-imidazol-4-ylethynyl]-pyridine. This composition is in the form of matrix pellets, and can be formed into tablets or incorporated in capsules. The present modified release formulations reduce CNS related adverse effects, improve therapeutic efficacy and improve tolerability.

The term "binder" refers to a substance used in the formulation of solid oral dosage forms to hold the active pharmaceutical ingredient and inactive ingredients together in a cohesive mix. Non-limiting examples of binders include gelatin, hydroxy propyl cellulose, hydroxy propyl methylcellulose, methylcellulose, polyvinyl pyrrolidone, sucrose, and starch.

The term "disintegrant" refers to an excipients which is added to a tablet or capsule blend to aid in the break up of the compacted mass when it is put into a fluid environment. Non limited examples of disintegrants include alginates, croscarmellose sodium, crospovidone, sodium starch glycolate, and pregelatinized starch.

The term "filler" refers to any pharmaceutical diluent.

The term "gel-forming cellulose ethers" refers to polymers derived by chemical modification of the natural polymer cellulose that is obtained from renewable botanical sources that formS a gel in aqueous medium under certain conditions

The term "glidant" refers to a substance that is added to a powder to improve its flowability. Nonlimiting examples of glidants include colloidal silicon dioxide, magnesium stearate, starch, and talc.

The term "hydrophilic polymers" refers to polymers that contain polar or charged functional groups, rendering them soluble in aqueous medium.

The term "insoluble polymer" refers to a polymer that is not soluble in aqueous medium.

The term "ionic polymer" refers to a polymer that consists of functional groups that are sensitive to pH. Depending on the pH, functional groups can ionize and help dissolve the polymer. "Anionic polymers" as used herein are generally soluble above about pH 5.

The term "lubricant" refers to an excipients which is added to a powder blend to prevent the compacted powder mass from sticking to the equipment during the tabletting or encapsulation process. It aids the ejection of the tablet form the dies, and can improve powder flow. Non-limiting examples of lubricants include calcium stearate, glycerine, hydrogenated vegetable oil, magnesium stearate, mineral oil, polyethylene glycol, and propylene glycol.

The term "matrix former" refers to a non-disintegrating polymer that provides the rigidity or we mechanical strength to the dosage form upon exposure to physiological fluid for controlling the release.

The term "modified-release" technology is the same as sustained-release (SR), sustained-action (SA), extended-release (ER, XR, or XL), timed-release, controlled-release (CR), and refers of a technology that provide release of a drug from a formulation over a defined period of time.

The term "multi-particulate composition" refers to solid particle systems employed in drug delivery systems including pellets, beads, millispheres, microspheres, microcapsules, aggregated particles, and others.

The term "particle size" refers to a measure of the diameter of the material as determined by laser diffraction.

The term "pH responding polymer" refers to ionizable polymers with pH dependent solubility that change permeability in response to changes in the gastrointestinal tract's physiological pH. Nonlimiting examples of pH responding polymers include hydroxypropylmethyl cellulose phthalate, cellulose acetate phthalate, cellulose acetate trimellitate, poly(meth)acrylates, and mixtures thereof. In one embodiment, poly(meth)acrylate.

The term "pharmaceutically acceptable," such as pharmaceutically acceptable carrier, excipient, etc. means pharmacologically acceptable and substantially non-toxic to the subject to which the particular compound is administered.

The term "pharmaceutically acceptable salt" refers to any salt derived from an inorganic or organic acid or base. Such salts include: acid addition salts formed with inorganic acids such as hydrochloric acid, hydrobromic acid, sulfuric acid, nitric acid, phosphoric acid; or formed with organic acids such as acetic acid, benzenesulfonic acid, benzoic, camphorsulfonic acid, citric acid, ethanesulfonic acid, fumaric acid, glucoheptonic acid, gluconic acid, glutamic acid, glycolic acid, hydroxynaphtoic acid, 2-hydroxyethanesulfonic acid, lactic acid, maleic acid, malic acid, malonic acid, mandelic acid, methanesulfonic acid, muconic acid, 2-naphthalenesulfonic acid, propionic acid, salicylic acid, succinic acid, tartaric acid, p-toluenesulfonic acid or trimethylacetic acid.

The term "plasticizer" refers to a substance that reduces the glass transition temperature of a polymer, making it more elastic and deformable, i.e. more flexible. Nonlimiting examples of plasticizers include dibutylsebacate, propylene glycol, triethylcitrate, tributylcitrate, castor oil, acetylated monoglycerides, acetyl triethylcitrate, acetyl butylcitrate, diethyl phthalate, dibutyl phthalate, triacetin, and medium-chain triglycerides.

The term "poorly soluble" refers to a compound whose solubility is below 33 mg/ml.

The term "rate controlling polymer" refers to pH independent, insoluble polymers that provide a pH independent permeability for drug release in a rate-controlling polymer membrane.

The term "release modifier" refers to any material that can change the dissolution rate of the active ingredient when added to the composition.

The term "spheronization enhancer" refers to a material added to the composition to enhance the sphericity of the particles in the composition.

The term "substantially water-soluble inert material" refers to any material that has a solubility in water greater than about 1 % w/w.

The term "surfactant" refers to a surface active compound that lowers the surface tension of a liquid and lowers the interfacial tension between two liquids, or between a liquid and a solid. Nonlimiting examples of surfactants include polysorbates and sodium lauryl sulfate.

The term "weakly basic" refers to compounds that are freely to moderately soluble at acidic pHs, but are poorly to practically insoluble at neutral and alkaline pHs, using the USP definitions for solubility.

The matrix pellet utilize a combination of pH responding enteric polymer and a rate controlling polymer as matrix components. The enteric polymer provides a pH microenvironment that results in a constant concentration gradient for drug diffusion through the matrix layer. After gastric transition, the pH increases from about 5.5 to about 7, resulting in a decrease in solubility of the basic compound 2-chloro-4-[1-(4-fluoro-phenyl)-2,5-dimethyl-1H-imidazol-4-ylethynyl]-pyridine. The pH responding polymer swells and dissolves, causing an increase in matrix porosity that compensates for the decrease in drug solubility, which enables a pH independent release rate.

The amount of the mGlu5 antagonist in the composition can vary from about 0.005 % to about 5 % by weight of the composition. In one embodiment, the amount of mGlu5 antagonist is from about 0.05 % to about 5 % by weight of the composition. In another embodiment the amount of mGlu5 antagonist is from about 0.005 % to about 0.5 % of the composition.

The particle size of the mGlu5 antagonist is ideally reduced to below 50 micron. In one embodiment the particle size of the compound is reduced to below 20 micron. In another embodiment, the particle size is reduced to below 10 micron (D90) for the mGlu5 antagonist.

### Active Ingredient

The active ingredient of the composition is the metabotropic glutamate 5 receptor (mGlu55) antagonists 2-chloro-4-[1-(4-fluoro-phenyl)-2,5-dimethyl-1H-imidazol-4-ylethynyl]-pyridine. This compound, their methods of manufacture, and therapeutic activity are described in commonly owned U.S. Patent Publication No. 2006-0030559, published February 9, 2006, and U.S. Patent No. 7,332,510, issued February 19, 2008.

In one embodiment, the metabotropic glutamate 5 receptor (mGlu55) antagonist comprises the compound 2-chloro-4-[1-(4-fluoro-phenyl)-2,5-dimethyl-1H-imidazol-4-ylethynyl]-pyridine.

Non-limiting examples of pharmaceutically acceptable salts are organic acid addition salts formed with acids, which form a physiological acceptable anion, for example, tosylate, methanesulfonate, maleate, malate, acetate, citrate, malonate, tartarate, succinate, benzoate, ascorbate, α-ketoglutarate, and α-glycerophosphate. Other pharmaceutically acceptable salts include inorganic salts, such as, for example, hydrochloride, sulfate, nitrate, bicarbonate, and carbonate salts. In one embodiment, the salt form of the mGlu5 antagonist of formula I exhibits low hygroscopic and good aqueous solubility. In another embodiment the salt is sulphate.

The compound 2-chloro-4-[1-(4-fluoro-phenyl)-2,5-dimethyl-1H-imidazol-4-ylethynyl]-pyridine has metabotropic glutamate 5 receptor (mGlu5) antagonist activity. It is useful for the treatment of CNS disorders, including, but not limited to, treatment-resistant depression (TRD) and Fragile X Syndrome.

The compound 2-chloro-4-[1-(4-fluoro-phenyl)-2,5-dimethyl-1H-imidazol-4-ylethynyl]-pyridine has two weakly basic moieties with pKa values of 4.64 and approximately 2. The compound is very lipophilic with a clog P value of 3.71 and a log D at pH 7.4 of greater than 3. The aqueous solubility of the free base is characterized by a steep pH-dependency with good solubility under acidic conditions (3.2 mg/ml at pH 1) and very low solubility at alkaline conditions (0.0003 mg./ml at pH 7). Because of this pH dependent solubility in physiological range, 2-chloro-4-[1-(4-fluoro-phenyl)-2,5-dimethyl-1H-imidazol-4-ylethynyl]-pyridine is classified as BCS class 2 compound.

Due to high solubility in gastric pH, conventional immediate release (IR) formulation of 2-chloro-4-[1-(4-fluoro-phenyl)-2,5-dimethyl-1H-imidazol-4-ylethynyl]-pyridine provides rapid release of the active ingredient once the formulation reaches the stomach. The peak plasma concentration occurs one hour following drug administration. However, the disadvantage of these IR formulations is that CNS-related adverse events, such as dizziness and somnolence, occur. These adverse events appear to be associated with high plasma peak or with the fast rise in plasma concentration that occurs after administration of the drug.

The present modified release formulation reduces CNS related adverse effects, improves therapeutic efficacy and improves tolerability,

### Matrix Pellets

In one embodiment, the composition comprises matrix pellets, where the drug is the mGlu5 antagonist 2-chloro-4-[1-(4-fluoro-phenyl)-2,5-dimethyl-1H-imidazol-4-ylethynyl]-pyridine, which is dispersed in the composition that is formed into pellets. The matrix pellets optionally can be coated with a further polymer layer and optionally encapsulated into capsules or compressed into tablets. In general, the drug and excipients are blended to form a uniform mixture. The mixture is then granulated to obtain a uniform matrix of drug and polymer. This consolidates the particle and improves flow. The granulated product is then extruded and then spheronized to form dense pellets having a spherical shape. The pellets then are dried to remove moisture.

The matrix pellet composition can be manufactured by methods known in the art, for example by extrusion spheronization, rotor granulation, spray drying, hot melt extrusion, top granulation and other standard technologies. In one embodiment, extrusion spheronization can be selected as the technology for manufacturing the matrix pellets. (See, e.g., Trivedi et al, Critical Reviews in Therapeutic Drug Carrier Systems, 24(1): 1-40 (2007); US Patent 6,004,996; and Issac-Ghebre-Selassi, et al., eds. Durgs and Pharmaceutical Sciences, Vol. 133, Pharmaceutical Extrusion Technology.)

Excipients can be used in the extrusion/spheronization process. These excipients can be selected based on functionality of the excipients. Non-limiting examples of types of excipients that can be used include fillers, binders, lubricants, disintegrants, surfactants, spheronization enhancers, glidants, and release modifiers. Some non-limiting examples of each of these types of excipients follows. Fillers can include, for example, calcium sulfate, dibasic calcium phosphate, lactose, mannitol, microcrystalline cellulose, starch, and sucrose. Binders can include, for example, gelatin, hydroxy propyl cellulose, hydroxy propyl methylcellulose, methylcellulose, polyvinyl pyrrolidone, sucrose, and starch. Lubricants can include, for example, calcium stearate, glycerine, hydrogenated vegetable oil, magnesium stearate, mineral oil, polyethylene glycol, and propylene glycol. Disintegrants can include, for example, alginates, croscarmellose sodium, crospovidone, sodium starch glycolate, and pregelatinized starch. Surfactants can include, for

example, polysorbates and sodium lauryl sulfate. Spheronization enhancers can include, for example, microcrystalline cellulose, microcrystalline, and cellulose/sodium-carboxymethyl cellulose. Glidants can include, for example, colloidal silicon dioxide, magnesium stearate, starch, and talc. Release modifiers can include, for example, ethyl cellulose, carnauba wax, and shellac.

In one embodiment, the matrix pellets contain MCC as a matrix former, HPMC as binder, and alternatively, an ionizable, pH responding polymer. The pH responding polymer can be any of those described above. In one embodiment, the pH responding polymer can be an ionic polymer, such as a poly(meth)acrylate like Eudragit L100-55^{®}. As discussed above, such pH responding polymers overcome the pH dependency of drug release for weakly basic compounds, such as those used in the described matrix pellets. As with the matrix tablet, the pH responding polymer creates a pH micro-environment that provides a constant concentration gradient for drug diffusion through the matrix or gel layer of the matrix pellet. After gastric transition, the pH increases to from about 5.5 to about 7 and the solubility of the basic mGlu5 antagonist decreases. In response to these conditions, the pH-responding enteric polymer swells and dissolves causing an increase in matrix porosity that compensates the decrease in drug solubility and enables a pH independent release rate.

The amount of pH responding polymer in the composition can vary from about 5 % to about 50 % by weight of the composition. In one embodiment, the pH responding polymer can be present in an amount from about 10 % to about 40 % by weight of the composition. In another embodiment, the pH responding polymer can be present in an amount from about 25 % to about 35 % of the composition. The composition exhibits an *in-vitro* release profile with NMT 70 % in one hour, NMT 85 % in 4 hour, and NLT 80 % in 8 hours.

In one embodiment, the particle size of the matrix pellet comprising the mGlu5 antagonist is ideally below about 3000 microns. In another embodiment, the particle size of the pellet is below about 2000 microns. In yet another embodiment, the average particle size of the pellet is about 400 microns to about 1500 microns.

The pH responding polymer also can be an insoluble polymer and can be used in combination with or without hydrophilic polymers. The mechanism of drug release for a matrix pellet containing an insoluble polymer is to modulate the permeability of the matrix. The aqueous fluids, e.g. gastrointestinal fluids, penetrate and dissolve the drug, which can then diffuse out from the matrix. Examples of insoluble polymers include, but are not limited to, ethyl cellulose (EC), polyvinylacetate (Kollidon SR^{®}), and polyvinylacetate/povidone copolymer. In one embodiment, ethyl cellulose (EC) or polyvinylacetate can be used to prepare the matrix pellets. In another embodiment, polyvinylacetate can be used to prepare the matrix pellets.

The amount of insoluble polymer in the composition can vary from about 5 % to about 50 % by weight of the composition. In one embodiment, the insoluble polymer can be present in an amount from about 10 % to about 3 5% by weight. In another embodiment, the insoluble polymer can be present in an amount from about 5 % to about 25 % of the composition. The composition exhibits an *in-vitro* release profile with NMT 70 % in one hour, NMT 85 % in 4 hour, and NLT 80 % in 8 hours.

The following example demonstrate the method manufacturing the compositions described herein and comparative examples of conventional modified release tablets.

### Example 1: Preparation of a modified release matrix pellet containing a pH responding polymer, MCC and sodium CMC mixture (F3)

Step 1: A preblended weighed amount of Avicell RC591^{®} (∼173 g) and Eudragit L100-55^{®} (75 g) were blended in a Turbula^{®} blender at 46 rpm for 5 minutes.
Step 2: 2-Chloro-4-[1-(4-fluoro-phenyl)-2,5-dimethyl-1H-imidazol-4-ylethynyl]-pyridine powder (1.6 g) and the polymer blend from Step 1 were mixed in a 1:1 ratio at 46 rpm for 5 minutes. Step 2 was repeated four times with portions of the polymer blend from Step 1. The resulting blend was sieved through 1.0 mm screen, and the screen was rinsed with the remaining polymer blend from Step 1 and blended for another 5 minutes. The blended material was transferred into a Dyazna^{®} vertical high shear granulator. All of the components were mixed for three minutes at a speed of 350 rpm (screw) and 1350 rpm (chopper). After blending for three minutes, the powder mixture was granulated by spraying purified water at 16 g/minute onto the powder mixer in the high shear granulator while continually mixing the contents using an impeller at 350 rpm and the chopper at 1350 rpm until a consistent granulation was obtained. The obtained wet granules were extruded through an LCI Xtruder^{®} Extruder using Screen # 1.0 mm and a speed setting of 40 rpm. The extruded material was transferred into an LUWA^{®} Marumerizer-Spheronizer and spheronized for 5 minutes at 1330 rpm. The spheronized material was collected and dried in a Vector FLM1^{®} fluid bed dryer with an inlet temperature of 60 °C and an air volume of 65 CFM for 1 hour. Using the weight of the obtained pellets, Talc (External component) was weighed and the amount adjusted. The talc was then mixed with the pellets for 5 minutes. The pellets were then filled into #0 opaque white non print gelatin capsules.

| **Compositions (mg) (1 mg dose)** | **Excipient Functionality** | **Matrix pellets** |
|---|---|---|
| 2-Chloro-4-[1-(4-fluoro-phenyl)-2,5-dimethyl-1H-imidazol-4-ylethynyl] -pyridine | Active ingredient | 1.3 |
| Avicel RC591^{®} (MCC and Sodium CMC blend) | Rate controlling and matrix forming polymer (MCC) and pH responding polymer (Sodium CMC) | 138.7 |
| Eudragit L100-55^{®} | pH responding polymer | 60.0 |
| Talc | Glidant | 3.2 |
| Total fill weight (mg) in a capsule | | 203.2 |

**Example 2: Modified release matrix pellets containing a pH responding polymer and microcrystalline cellulose**

| **Compositions (mg) (1 mg dose)** | **Excipient Functionality** | **Matrix pellets** |
|---|---|---|
| 2-Chloro-4-[1-(4-fluoro-phenyl)-2,5-dimethyl-1H-imidazol-4-ylethynyl]-pyridine | Active ingredient | 1.3 |
| Avicel 101^{®} | Rate controlling and matrix forming polymer | 128.2 |
| Eudragit L100-55^{®} | pH responding polymer | 60.0 |
| Pharmacoat 603^{®} | Binder | 10.0 |
| Talc | Glidant | 0.5 |
| Total fill weight (mg) in a capsule | | 203 |

Weighed the 2-Chloro-4-[1-(4-fluoro-phenyl)-2,5-dimethyl-1H-imidazol-4-ylethynyl]-pyridine powder (7.8 g) and Microcrystalline Cellulose (Avicel, PH-101; 769 g) were weighed, placed into a Turbula^{®} blender, and mixed for 30 minutes at 40 rpm. The contents were passed through a Fitz-mill^{®} Screen #3 with Knife forward speed of ∼2500 rpm. The milled material was transferred to VG-25^{®} high shear granulator and mixed with Eudragit L100-55^{®} (360 g) and Pharmacoat 603^{®} (60 g) at a speed of 250 rpm (screw) and 1500 rpm (chopper) for two minutes. After mixing for two minutes, water was added at a spray rate of 100 g/minutes until a consistent granulation was obtained. The wet granules were extruded through an LCI Xtruder Extruder^{®} using Screen # 1.0 mm and speed setting of 20 rpm. The extruded material was then transferred to a LUWA^{®} Marumerizer-Spheronizer and spheronized for 10 minutes at 1330 rpm. The spheronized material was collected and dried in a fluid bed dryer with an inlet temperature of 60 °C and an air volume of 60 CFM for 3 hours. Using the weight of the obtained pellets, Talc (External component) was weighed and the amount adjusted. The talc was then mixed with the pellets for 5 minutes. The pellets were then filled into #2 opaque white non print gelatin capsules.

## Claims

1. A pharmaceutical modified release composition comprising the compound 2-chloro-4-[1-(4-fluoro-phenyl)-2,5-dimethyl-1H-imidazol-4-ylethynyl]-pyridine and pharmaceutically acceptable salts thereof, an insoluble rate-controlling polymer, selected from the group consisting of polyvinylacetate, ethylcellulose, polyvinylacetate/povidone copolymers and microcrystalline cellulose, and containing a pH responding polymer, selected from the group consisting of ionic poly(meth)acrylates, and mixtures thereof, in the form of a matrix pellet composition, comprising 2-Chloro-4-[1-(4-fluoro-phenyl)-2,5-dimethyl-1H-imidazol-4-ylethynyl]-pyridine. dispersed within formed matrix pellets.

2. The composition according to claim 1, wherein the compound is present in an amount from 0.005 % to 5 % by weight.

3. The composition according to any one of claims 1 or 2 , wherein the compound is present in an amount from 0.5 % to 5 %.

4. The composition according to claim 1, wherein the particle size of the compound is 50 microns or less.

5. The composition according to any one of claim 1 or 4, wherein the particle size of the compound is 20 microns or less.

6. The composition according to any one of claims 1, 4 or 5 , wherein the particle size of the compound is 10 microns or less.

7. The composition according to claim 1, wherein the pH responding polymer is present in an amount from 5 % to 50 % by weight of the composition.

8. The composition according to any one of claims 1 and 7, wherein the pH responding polymer is present in an amount from 10 % to 40 % by weight of the composition.

9. The composition according to any one of claims 1, 7 or 8 wherein the pH responding polymer is present in an amount from 25 % to 35 % by weight of the composition.

10. The composition according to claim 1, wherein the matrix pellets have a particle size of less than 3000 microns.

11. The composition according to any one of claims 1 or 10, wherein the matrix pellets have a particle size of less than 2000 microns.

12. The composition according to any one of claims 1, 10 or 11, wherein the matrix pellets have an average particle size of from 400 microns to 1500 microns.

13. The composition according to claim 1, wherein the insoluble polymer is present in an amount from 5 % to 50 % by weight of the composition.

14. The composition according to any one of claims 1 or 13, wherein the insoluble polymer is present in an amount from 10 % to 35 % by weight of the composition.

15. The composition according to any one of claims 1, 13 or 14, wherein the insoluble polymer is present in an amount from 5 % to 2 5% by weight of the composition.

16. The composition according to claim 1, wherein the composition further comprises a filler, disintegrant, surfactant, glidant, lubricant spheronization enhancer, release modifier and/or binder.

17. A composition according to claim 1, which comprises
| **Compositions (mg) (1 mg dose)** | **Excipient Functionality** | **Matrix pellets** |
|---|---|---|
| 2-Chloro-4-[1-(4-fluoro-phenyl)-2,5-dimethyl-1H-imidazol-4-ylethynyl] -pyridine | Active ingredient | 1.3 |
| Avicel RC591^{®} (MCC and Sodium CMC blend) | Rate controlling and matrix forming polymer (MCC) and pH responding polymer (Sodium CMC) | 138.7 |
| Eudragit L100-55^{®} | pH responding polymer | 60.0 |
| Talc | Glidant | 3.2 |
| Total fill weight (mg) in a capsule | | 203.2 |

18. A composition according to claim 1, which comprises
| **Compositions (mg) (1 mg dose)** | **Excipient Functionality** | **Matrix pellets** |
|---|---|---|
| 2-Chloro-4-[1-(4-fluoro-phenyl)-2,5-dimethyl-1H-imidazol-4-ylethynyl]-pyridine | Active ingredient | 1.3 |
| Avicel 101^{®} | Rate controlling and matrix forming polymer | 128.2 |
| Eudragit L100-55^{®} | pH responding polymer | 60.0 |
| Pharmacoat 603^{®} | Binder | 10.0 |
| Talc | Glidant | 0.5 |
| Total fill weight (mg) in a capsule | | 203 |

## Patentansprüche

1. Ein Arzneimittel mit modifizierter Freisetzung, umfassend die Verbindung 2-Chlor-4-[1-(4-fluor-phenyl)-2,5-dimethyl-1H-imidazol-4-ylethinyl]-pyudin und pharmazeutisch verträgliche Salze davon, ein unlösliches geschwindigkeitsbestimmendes Polymer, ausgewählt aus der Gruppe bestehend aus Polyvinylacetat, Ethylcellulose, Polyvinylacetat/Povidon-Copolymeren und mikrokristalliner Cellulose, und enthaltend ein pH-empfindliches Polymer, ausgewählt aus der Gruppe bestehend aus ionischen Poly(meth)acrylaten und Gemische davon, in Form einer Matrixpelletzusammensetzung, welche 2-Chlor-4-[1-(4-fluor-phenyl)-2,5-dimethyl-1H-imidazol-4-ylethinyl]-pyridin dispergiert innerhalb der gebildeten Matrixpellets umfasst.

2. Die Zusammensetzung gemäß Anspruch 1, wobei die Verbindung in einer Menge von 0,005 Gew.-% bis 5 Gew.-% vorliegt.

3. Die Zusammensetzung gemäß einem der Ansprüche 1 oder 2, wobei die Verbindung in einer Menge von 0,5% bis 5% vorliegt.

4. Die Zusammensetzung gemäß Anspruch 1, wobei die Teilchengröße der Verbindung 50 Mikrometer oder weniger beträgt.

5. Die Zusammensetzung gemäß einem der Ansprüche 1 oder 4, wobei die Teilchengröße der Verbindung 20 Mikrometer oder weniger beträgt.

6. Die Zusammensetzung gemäß einem der Ansprüche 1, 4 oder 5, wobei die Teilchengröße der Verbindung 10 Mikrometer oder weniger beträgt.

7. Die Zusammensetzung gemäß Anspruch 1, wobei das pH-empfindliche Polymer in einer Menge von 5 Gew.-% bis 50 Gew.-% der Zusammensetzung vorliegt.

8. Die Zusammensetzung gemäß einem der Ansprüche 1 und 7, wobei das pH-empfindliche Polymer in einer Menge von 10 Gew.-% bis 40 Gew.-% der Zusammensetzung vorliegt.

9. Die Zusammensetzung gemäß einem der Ansprüche 1, 7 oder 8, wobei das pH-empfindliche Polymer in einer Menge von 25 Gew.-% bis 35 Gew.-% der Zusammensetzung vorliegt.

10. Die Zusammensetzung gemäß Anspruch 1, wobei die Matrixpellets eine Teilchengröße von weniger als 3000 Mikrometer aufweisen.

11. Die Zusammensetzung gemäß einem der Ansprüche 1 oder 10, wobei die Matrixpellets eine Teilchengröße von weniger als 2000 Mikrometer aufweisen.

12. Die Zusammensetzung gemäß einem der Ansprüche 1, 10 oder 11, wobei die Matrixpellets eine mittlere Teilchengröße von 400 Mikrometer bis 1500 Mikrometer aufweisen.

13. Die Zusammensetzung gemäß Anspruch 1, wobei das unlösliche Polymer in einer Menge von 5 Gew.-% bis 50 Gew.-% der Zusammensetzung vorliegt.

14. Die Zusammensetzung gemäß einem der Ansprüche 1 oder 13, wobei das unlösliche Polymer in einer Menge von 10 Gew.-% bis 35 Gew.-% der Zusammensetzung vorliegt.

15. Die Zusammensetzung gemäß einem der Ansprüche 1, 13 oder 14, wobei das unlösliche Polymer in einer Menge von 5 Gew.-% bis 25 Gew.-% der Zusammensetzung vorliegt.

16. Die Zusammensetzung gemäß Anspruch 1, wobei die Zusammensetzung ferner ein Füllmittel, Sprengmittel, oberflächenaktives Mittel, Gleitmittel, Schmiermittel Sphäronisationsverstärker, Freisetzungsmodifikator und/oder Bindemittel umfasst.

17. Eine Zusammensetzung gemäß Anspruch 1, umfassend
| **Zusammensetzungen (mg) (1-mg-Dosis)** | **Funktionalität des Exzipienten** | **Matrixpellets** |
|---|---|---|
| 2-Chlor-4-[1-(4-fluor-phenyl)-2,5-dimethyl-1H-imidazol-4-ylethinyl]-pyridin | Wirkstoff | 1,3 |
| Avicel RC591^{®} (MCC und Natrium-CMC-Mischung) | Geschwindigkeitsbestimmendes und matrixbildendes Polymer (MCC) und pH-empfindliches Polymer (Natrium-CMC) | 138,7 |
| Eudragit L100-55^{®} | pH-empfindliches Polymer | 60,0 |
| Talk | Gleitmittel | 3,2 |
| Gesamtfüllgewicht (mg) in einer Kapsel | | 203,2 |

18. Eine Zusammensetzung gemäß Anspruch 1, umfassend
| **Zusammensetzungen (mg) (1-mg-Dosis)** | **Funktionalität des Exzipienten** | **Matrixpellets** |
|---|---|---|
| 2-Chlor-4-[1-(4-fluor-phenyl)-2,5-dimethyl-1H-imidazol-4-ylethinyl]-pyridin | Wirkstoff | 1,3 |
| Avicel 101^{®} | Geschwindigkeitsbestimmendes und matrixbildendes Polymer | 128,2 |
| Eudragit L100-55^{®} | pH-empfindliches Polymer | 60,0 |
| Pharmacoat 603^{®} | Bindemittel | 10,0 |
| Talk | Gleitmittel | 0,5 |
| Gesamtfüllgewicht (mg) in einer Kapsel | | 203 |

## Revendications

1. Composition pharmaceutique à libération modifiée comprenant le composé 2-chloro-4-[1-(4-fluoro-phényl)-2,5-diméthyl-1H-imidazol-4-yléthynyl]-pyridine et ses sels phaimaceutiquement acceptables, un polymère insoluble de contrôle de taux, choisi dans le groupe consistant en polyacétate de vinyle, éthylcellulose, copolymères de polyacétate de vinyle/povidone et cellulose microcristalline, et contenant un polymère sensible au pH, choisi dans le groupe consistant en poly(méth)acrylates ioniques, et leurs mélanges, sous la forme d'une composition de granulés matriciels, comprenant la 2-chloro-4-[1-(4-fluoro-phényl)-2,5-diméthyl-1H-imidazol-4-yléthynyl]-pyridine, dispersée au sein des granulés matriciels formés.

2. Composition selon la revendication 1, dans laquelle le composé est présent dans une quantité de 0,005 % à 5 % en poids.

3. Composition selon l'une quelconque des revendications 1 ou 2, dans laquelle le composé est présent dans une quantité de 0,5 % à 5 %.

4. Composition selon la revendication 1, dans laquelle la taille des particules du composé est de 50 microns ou inférieure.

5. Composition selon l'une quelconque des revendications 1 ou 4, dans laquelle la taille des particules du composé est de 20 microns ou inférieure.

6. Composition selon l'une quelconque des revendications 1, 4 ou 5, dans laquelle la taille des particules du composé est de 10 microns ou inférieure.

7. Composition selon la revendication 1, dans laquelle le polymère sensible au pH est présent dans une quantité de 5 % à 50 % en poids de la composition.

8. Composition selon l'une quelconque des revendications 1 et 7, dans laquelle le polymère sensible au pH est présent dans une quantité de 10 % à 40 % en poids de la composition.

9. Composition selon l'une quelconque des revendications 1, 7 ou 8, dans laquelle le polymère sensible au pH est présent dans une quantité de 25 % à 35 % en poids de la composition.

10. Composition selon la revendication 1, dans laquelle les granulés matriciels ont une taille de particule inférieure à 3 000 microns.

11. Composition selon l'une quelconque des revendications 1 ou 10, dans laquelle les granulés matriciels ont une taille de particule inférieure à 2 000 microns.

12. Composition selon l'une quelconque des revendications 1, 10 ou 11, dans laquelle les granulés matriciels ont une taille de particule de 400 microns à 1 500 microns.

13. Composition selon la revendication 1, dans laquelle le polymère insoluble est présent dans une quantité de 5 % à 50 % en poids de la composition.

14. Composition selon l'une quelconque des revendications 1 ou 13, dans laquelle le polymère insoluble est présent dans une quantité de 10 % à 35 % en poids de la composition.

15. Composition selon l'une quelconque des revendications 1, 13 ou 14, dans laquelle le polymère insoluble est présent dans une quantité de 5 % à 25 % en poids de la composition.

16. Composition selon la revendication 1, la composition comprenant en outre une charge, un agent de désagrégation, un tensioactif, un agent de glissement, un lubrifiant, un amplificateur de sphéronisation, un modificateur de la libération et/ou un liant.

17. Composition selon la revendication 1, qui comprend
| Compositions (mg) (dose de 1 mg) | Fonctionnalité de l'excipient | Pellets matriciels |
|---|---|---|
| 2-chloro-4-[1-(4-fluoro-phényl)-2,5-diméthyl-1H-imidazol-4-yléthynyl]-pyridine | Principe actif | 1,3 |
| Avicel RC591® (mélange de MCC et de CMC sodique) | Polymère de contrôle de taux et de formation de matrice (MCC) et polymère sensible au pH (CMC sodique) | 138,7 |
| Eudragit L100-55® | Polymère sensible au pH | 60,0 |
| Talc | Agent de glissement | 3,2 |
| Poids de remplissage total (mg) dans une capsule | | 203,2 |

18. Composition selon la revendication 1, qui comprend
| Compositions (mg) (dose de 1 mg) | Fonctionnalité de l'excipient | Pellets matriciels |
|---|---|---|
| 2-chloro-4-[1-(4-fluoro-phényl)-2,5-diméthyl-1H-imidazol-4-yléthynyl]-pyridine | Principe actif | 1,3 |
| Avicel 101® | Polymère de contrôle de taux et de formation de matrice | 128,2 |
| Eudragit L100-55® | Polymère sensible au pH | 60,0 |
| Phaimacoat 603® | Liant | 10,0 |
| Talc | Agent de glissement | 0,5 |
| Poids de remplissage total (mg) dans une capsule | | 203 |
